# EUROPEAN PATENT SPECIFICATION

(11) **EP 3 919 029 B1**
(45) Date of publication and mention of the grant of the patent: **03.07.2024**
(21) Application number: 20748785.1
(22) Date of filing: 28.01.2020
(51) Int. Cl.: A61F 2/82, A61F 2/915, A61L 31/02, A61L 31/12, A61L 31/08, A61L 31/16

(54) **BIOABSORBABLE STENT**
BIORESORBIERBARER STENT
ENDOPROTHÈSE BIOABSORBABLE

(30) Priority: 30.01.2019 JP 2019014434; 28.03.2019 JP 2019062873; 08.01.2020 JP 2020001519
(43) Date of publication of application: 08.12.2021
(73) Proprietor: JAPAN Medical Device Technology Co., Ltd., Kamimashiki-gun, Kumamoto 861-2202 (JP)
(72) Inventor: YAMASHITA, Shuzo, Kamimashiki-gun, Kumamoto 861-2202 (JP); SASAKI, Makoto, Kamimashiki-gun, Kumamoto 861-2202 (JP); WADA, Akira, Kamimashiki-gun, Kumamoto 861-2202 (JP)
(74) Representative: Pact-IP
(86) International application number: PCT/JP2020/003050
(87) International publication number: WO 2020/158761

(56) References cited:
- WO-A1-2018/139647
- WO-A1-2019/182003
- WO-A1-2020/012529
- JP-A- 2007 195 883
- ZHANG Y et al.: "Characterization and degradation comparison of DLC film on different magnesium alloys.", Surfaces & Coatings Technology, vol. 205, no. 1, 2010, pages S15-S20, XP027556841,
- Yan Lin Wei; Lin Lin Huang; Li Jun Han; Ya Shao Chen: "Corrosion resistance and surface biocompatibility of diamond-like carbon coating on AZ31D magnesium alloy", International Journal of Surface Science and Engineering, vol. 10, no. 2, 30 November 2015 (2015-11-30), pages 101-115, XP009529458, CH ISSN: 1749-785X, DOI: 10.1504/IJSURFSE.2016.076508

## Description

### CROSS REFERENCE TO THE RELATED APPLICATION

This application is based on and claims Convention priority to Japanese Patent Application No. 2019-014434, filed January 30, 2019, Japanese Patent Application No. 2019-062873, filed March 28, 2019, and Japanese Patent Application No. 2020-001519 filed January 8, 2020.

The present invention relates to a bioabsorbable stent that is implanted in a stenosis part or an occlusion part, especially in the coronary arteries, in lumen of living body so as to keep the inserted part open, and to be gradually degraded in the living body.

### BACKGROUND ART

The ischemic heart diseases (myocardial infarction, angina, etc.) caused by stenosis and occlusion of the coronary arteries are critical diseases which disturb supply of the blood (nutrition, oxygen, etc.) to a cardiac muscle, and are mentioned to the second place of the cause of Japanese death. As medical treatment of these disease, there have been widely used surgeries with low invasiveness using a catheter (percutaneous transluminal coronary angioplasty), not a surgical operation to open chest part (coronary-arteries bypass surgery). Especially, since a coronary-arteries stent placement has a small recurrence rate of stenosis (re-stenosis) compared with the conventional balloon formation, the stent replacement is regarded as the most promising remedy.

However, although coronary-arteries stent surgery gains popularity nowadays, there have been still many cases to cause complications at a certain period of postoperative time. The reason for this is considered that the stent made of cobalt chrome alloy body or stainless-steel body remains in the affected part with allowing intravascular wall open after being placed, so that the stent suppresses original blood vessel movement (pulsation), and continuously gives mechanical and chemical stimuli to the intravascular wall. In the medical front line, there has been expanding expectation for bioabsorbable stents as new medical equipment to solve the problem, i.e., the bioabsorbable stent having validity and safety to medical treatment for ischemic heart disease while enabling recovery of blood vessel movement after a certain period of postoperative time. A bioabsorbable stent is sometimes called as a bioabsorbable scaffold in recent years. Likewise, the bioabsorbable stent described here means a bioabsorbable scaffold.

Since the bioabsorbable stent has the innovative function to self-decompose gradually through the recovery process of the affected part, the bioabsorbable stent is a promising device being capable of cancelling the above-mentioned stimuli at an early stage, and making the affected part to regain normal blood vessel movement. This function is also advantageous to shorten a dosing period of antiplatelet agent for preventing complications, as well as to enhance the flexibility of the choice in postoperative re-medical treatment.

On the other hand, the bare metal stent made of a bioabsorbable magnesium alloy body has a problem that mechanical strength is spoiled immediately during expansion in an aqueous solution because of acceleration of decomposition (corrosion) throughout the surface where water molecules are in contact. Accordingly, such a bioabsorbable magnesium stent has difficulty in practical application. The decomposition rate of a magnesium alloy in the living body environment is much faster than that of a polylactic acid. Considering the requirement to maintain sufficient blood vessel bearing power (radial force) for 1 to 6 months after stent implant, the bioabsorbable magnesium has by no means suitable characteristics.

Patent Document 1 discloses a method for suppressing corrosion of a magnesium alloy body containing aluminum and rare earth metal with a waterproof barrier. The method includes forming a magnesium fluoride layer on the surface of the magnesium alloy body, and then further forming chemical conversion film layers of aluminum oxide (Al₂O₃) and a poly(aluminum ethylene glycol) polymer (alucone) on the magnesium fluoride layer because single magnesium fluoride layer is not sufficiently capable of having the corrosion of the material delayed. Also Patent Document 2 shows a bioabsorbable stent comprising a core structure of a magnesium alloy, the stent comprising a first anticorrosive layer containing magnesium fluoride as a main component formed on the core structure.

### PATENT DOCUMENTS

Patent Document 1 US2016/0129162A1
Patent Document 2 WO 2018/139647 A1

### SUMMARY OF THE INVENTION

### PROBLEMS TO BE SOLVED BY THE INVENTION

Although Patent Document 1 describes to form a magnesium fluoride layer on the surface of the magnesium alloy as a barrier layer for delaying the corrosion of the magnesium alloy, and further chemical conversion film layers of aluminum oxide (Al₂O₃) and a poly(aluminum ethylene glycol) polymer (alucone) on the magnesium fluoride layer, aluminum has a problem in terms of safety to the human body. Accordingly, it is desirable to control corrosiveness of the magnesium alloy with an aluminum-free treatment agent.

An object of the present invention is to provide a bioabsorbable magnesium alloy stent that has a controlled corrosiveness of the magnesium alloy, is good at deformation followability, and comprises a coating layer with highly safe for the human body by forming a magnesium fluoride layer (first anticorrosive layer) as an anticorrosive layer on the surface of a magnesium alloy, and further a carbon-coated layer (second anticorrosive layer) made of a diamond-like carbon (DLC) preferably a silicon-containing diamond-like carbon (Si-DLC) on the magnesium fluoride layer.

In order to achieve the above-mentioned object, as a result of intensive study for surface treatment of the magnesium alloy constituting a stent body, the inventors of the present invention have reached the present invention.

That is, the present invention includes the following aspects.

### Aspect 1

A bioabsorbable stent comprising a core structure of a magnesium alloy, a first anticorrosive layer containing magnesium fluoride as a main component formed on the core structure, and a second anticorrosive layer of a carbon-coated layer containing a diamond-like carbon on the first anticorrosive layer, the first anticorrosive layer has a layer thickness of 0.1 to 3 µm and the second anticorrosive layer has a layer thickness of 10 nm to 5 µm.

### Aspect 2

A method for producing a bioabsorbable stent comprising a magnesium alloy containing (1) fluorinating a surface of a core structure made of a magnesium alloy, to form a first anticorrosive layer containing magnesium fluoride as a main component and having a layer thickness of 0.1 to 3 µm, and then, (2) subjecting the core structure with the first anticorrosive layer to be placed in a high-frequency plasma CVD apparatus such that a diamond-like carbon film is coated on the core structure via introduction of a carbon-containing source gas so as to form a second anticorrosive layer having a layer thickness of 10 nm to 5 µm.

### EFFECT OF THE INVENTION

According to aspect 1 of the present invention, desired corrosion resistance can be imparted to a magnesium alloy constituting a stent structure by forming a first anticorrosive layer containing magnesium fluoride as a main component on the surface of the magnesium alloy and then forming a second anticorrosive layer comprising a diamond-like carbon coat layer on the first anticorrosive layer. The stent having the above constituents can sustain mechanical strength in an expanded state in a simulated plasma solution (EMEM + 10% FBS) at 37°C under 5% CO₂ atmosphere at least 1 week, preferably over one month.

Since the first anticorrosion layer containing magnesium fluoride as a main component has biodegradability, direct contact of body fluid with the first anticorrosion layer causes acceleration of biodegradation of the first anticorrosion layer containing magnesium fluoride layer as the main component, resulting in insufficient corrosion resistance due to disappearance of the first anticorrosion layer. However, the second anticorrosion layer that comprises a diamond-like carbon layer and covers the first anticorrosion layer achieves synergistic effect of two layers to improve corrosion resistance of the first anticorrosion layer for a longer period of time. Without the first anticorrosion layer, providing a thicker second anticorrosion layer that comprises the diamond-like carbon layer alone is not effective because the unbiodegradable diamond-like carbon requires an enhanced amount of diamond-like carbon embedded in the blood vessel surface so as to lead to higher possibility of cracks during expansion.

Although there is a concern about safety to the human body in the stent structure disclosed in Patent Document 1 because the stent structure comprises an anticorrosion layer made of magnesium fluoride and a chemical conversion coating layer containing aluminum [aluminum oxide layer and poly (aluminum ethylene glycol) (alucone)] formed on the anticorrosion layer, the present invention provides a stent structure with safety to human body because of the anticorrosion layer excluding aluminum.

The magnesium alloy can be composed of substantially single-phase solid solution or have a microstructure in which nanometer-sized fine Zr-bearing precipitates are dispersed in the single-phase alloy. The magnesium alloy has excellent deformability (ductility, elongation ability) because of its fine and uniform particle size and has excellent mechanical properties such as tensile strength and proof strength because of the absence of coarse precipitates at which a fracture starts.

Where the unavoidable impurities of the magnesium alloy include Fe, Ni, Co, and/or Cu, a content of each of Fe, Ni, Co, and Cu being preferably lower than 10 ppm. The magnesium alloy may preferably be free of Co as an unavoidable impurity.

Since the above-mentioned magnesium alloy has an excellent deformation property, the stent made of the magnesium alloy can stably maintain the shape thereof in blood vessels, as well as suitably control the biodegradability thereof.

The above-mentioned magnesium alloy is excellent in safety to the human body because the magnesium alloy is free of a rare earth element and an aluminum.

The first and second anticorrosive layers may be formed over the entire surface of the core structure enable to prevent progress of local corrosion.

The thickness of the first anticorrosion layer is 0.1 µm or more and 3 µm or less. Too thin thickness may cause poor anticorrosion, while too thick thickness may lead to insufficient deformation followability.

The diamond-like carbon in the second anticorrosive layer may be a diamond-like carbon containing silicon, in comparison with the diamond-like carbon coat layer containing no element other than carbon, the diamond-like carbon containing silicon layer has improved adhesive property to the Mg alloy surface constituting coating layer so as to impart deformation followability.

The thickness of the diamond-like carbon layer (including the silicon-containing diamond-like carbon coat layer) is 10 nm or more and 5 µm or less, preferably from 20 nm to 2 µm, and more preferably from 20 nm to 500 nm. Such a thickness exhibits anticorrosion effect and prevents the stent from generating cracks during deformation.

It is preferable that a biodegradable polymer layer is formed on the surface of at least a part of the second anticorrosive layer. The biodegradable polymer layer enables smooth insertion of the stent into blood vessels. Also, the biodegradable polymer layer may contain a medicine (such as a limus type intimal thickening inhibitor).

The biodegradable polymer layer may be composed of two layers, a first layer on the side of the second anticorrosive layer and a second layer on the side of blood. A medicine may be contained in any one of the first and second layers, or both layers.

According to the second aspect of the present invention, by coating the diamond-like carbon film layer as the second anticorrosive layer on the surface of the first anticorrosive layer of the bioabsorbable stent, it is possible to produce a bioabsorbable stent having a first anticorrosive layer and a second anticorrosive layer.

The present invention will be more clearly understood from the following description of preferred embodiments thereof, when taken in conjunction with the accompanying drawings. However, the embodiments and the drawings are given only for the purpose of illustration and explanation, and are not to be taken as limiting the scope of the present invention in any way whatsoever, which scope is to be determined by the appended claims.
Fig. 1 shows a schematic view illustrating constituents of a stent according to the present invention;
Fig. 2 shows a plan view illustrating an example of a scaffold structure of a stent according to the present invention;
Fig. 3 shows a plan view illustrating another example of a scaffold structure of a stent according to the present invention;
Fig. 4 shows a schematic cross-sectional view illustrating an example of an apparatus for forming a second anticorrosive layer.

### DESCRIPTION OF THE EMBODIMENTS

### Basic Structure of Stent

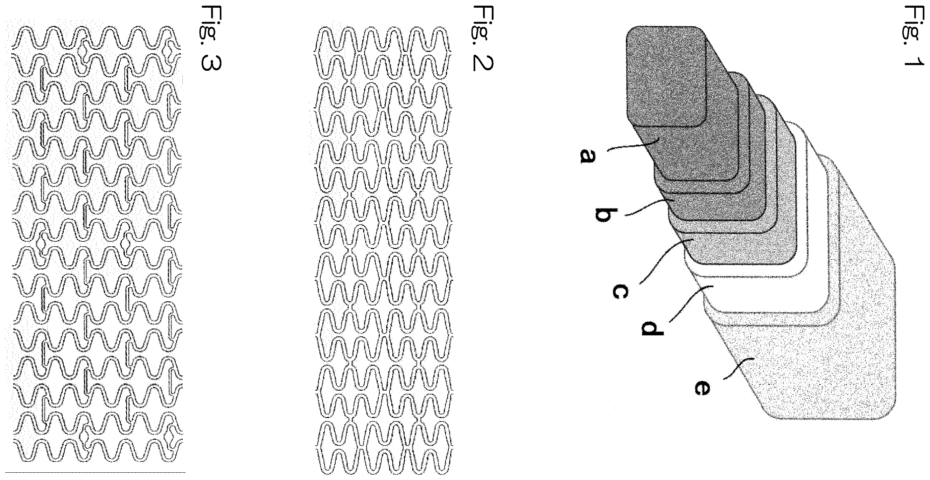

As shown in Fig. 1, an example of a stent of the present invention comprises: a core structure **(a)** comprising a magnesium alloy (Mg alloy); a first anticorrosive layer **(b)** formed on an entire surface of the core structure **(a)** and comprising magnesium fluoride (MgF₂) [the first anticorrosive layer contains Mg(OH)₂ etc. formed by oxidation of Mg on the layer surface and thus exhibits hydrophilicity]; a second anticorrosive layer **(c)** of a carbon-coated layer formed on the first anticorrosive layer **(b)** and comprising diamond-like carbon (preferably silicon-containing diamond-like carbon); a biodegradable resin layer **(d)** formed at least a part of a surface of the second anticorrosive layer **(c)**; and a biodegradable resin layer **(e)** formed on the biodegradable resin layer **(d)** and containing a medicine or a drug (it should be noted that the biodegradable resin layer **(d)** may contain a medicine or a drug, instead of providing a biodegradable resin layer **(e)** containing a medicine).

The following technical elements are provided to obtain the above configuration: an element for selecting a composition of the magnesium alloy for constituting the core structure having a biodegradability and excellent deformability; an element for forming the first anticorrosive layer containing MgFz as a main component over the entire surface of the core structure so as to control corrosion of the core structure comprising the selected magnesium alloy; an element for forming the second anticorrosive layer of a carbon-coated layer comprising diamond-like carbon (preferably silicon-containing diamond-like carbon) on the first anticorrosive layer; and optionally an element for forming a bioabsorbable material layer coated on the core structure and containing a medicine or a drug.

### Magnesium Alloy

The core structure of the stent according to the present invention comprises a bioabsorbable magnesium alloy.

In the present invention, the core structure of the stent comprises a magnesium alloy that contains 90 wt% or more of magnesium (Mg) as a main component; and zinc (Zn), zirconium (Zr), and manganese (Mn) as accessary components and is free of aluminum (Al) and at least one rare earth element(s) selected from the group consisting of scandium (Sc), Yttrium (Y), dysprosium (Dy), samarium (Sm), cerium (Ce), gadolinium (Gd), lantern (La), neodymium (Nd), and 30 ppm or less of unavoidable impurity selected from the group consisting of iron (Fe), nickel (Ni), cobalt (Co) and copper (Cu). Such composition enables to ensure safety to the human body and mechanical properties.

In order to enhance safety to the human body and mechanical properties, the content of Mg may more suitably be 93 wt% or more and further suitably be 95 wt% or more.

Absence of at least one rare earth element selected from the group consisting of scandium (Sc), yttrium (Y), dysprosium (Dy), samarium (Sm), cerium (Ce), gadolinium (Gd), and lantern (La) preferably all of the rare earth element(s), as well as absence of aluminum can prevent a potential harmful effect to the human body.

A magnesium alloy of the present invention contains, in % by mass, 0.95 to 2.00 % of Zn, 0.05 % or more and less than 0.30 % of Zr, 0.05 to 0.20 % of Mn, and the balance consisting of Mg and unavoidable impurities, wherein the magnesium alloy has a particle size distribution with an average crystal particle size from 1.0 to 3.0 µm and a standard deviation of 0.7 µm or smaller.

The present invention has revealed that controlling the composition of the magnesium alloy within the above range improves plastic workability, and that finer and more uniform particle size of the alloy improves the properties such as elongation at break.

The magnesium alloy having the above features can avoid formation of coarse precipitates which may be triggers (starting points) of fractures and thereby reduce the possibility of breakage during and after deformation. It should be noted that although Zr, which is added in order to reduce the crystal particle size of the alloy, may form precipitates, the precipitates are typically dispersed at a nanometer scale (in a size smaller than 100 nm) in the matrix phase and thus has a negligible impact on deformation and corrosion of the alloy.

### Zinc (Zn): in % by mass, 0.95 % or more and 2.00 % or less

Zn is added in order to enhance the strength and elongation ability of the alloy by forming a solid solution with Mg. Where the content of Zn is less than 0.95 %, a desired effect cannot be obtained. An amount of Zn exceeding 2.00 % is not preferred because such an amount may exceed a solid solubility limit of Zn in Mg so that Zn-rich precipitates are formed, resulting in reduced corrosion resistance. For this reason, Zn content is regulated to 0.95 % or more and 2.00 % or less. The content of Zn may be less than 2.00 %.

### Zirconium (Zr): in % by mass, 0.05 % or more and less than 0.30 %

Zr hardly forms a solid solution with Mg and forms fine precipitates, providing an effect of preventing formation of coarse crystal particles of the alloy. Addition of Zr at an amount less than 0.05 % cannot provide a sufficient effect. Addition of Zr at an amount equal to or exceeding 0.30 % leads to formation of a large amount of precipitates, with a reduced effect of particle size reduction. In addition, corrosion and breakage would start occurring at portions where the precipitates are biased. For this reason, content of Zr is regulated to 0.05 % or more and less than 0.30 %. The content of Zr may be 0.10 % or more and less than 0.30 %.

### Manganese (Mn): in % by mass, 0.05 % or more and 0.20 % or less

Mn allows the alloy to have extremely fine particle size and have improved corrosion resistance. Where an amount of Mn is less than 0.05 %, a desired effect cannot be obtained. An amount of Mn exceeding 0.20 % is not preferred because plastic workability of the alloy tends to decrease. For this reason, Mn content is regulated to 0.05 % or more and 0.20 % or less. A preferable content of Mn may be 0.10 % or more and 0.20 % or less.

### Unavoidable Impurities

Preferably, the content of unavoidable impurities is also controlled in the magnesium alloy for medical use. Since Fe, Ni, Co, and Cu promote corrosion of the magnesium alloy, the content of each of these unavoidable impurities is preferably lower than 10 ppm, further preferably 5 ppm or lower, and preferably substantially absent. The total content of the unavoidable impurities is preferably 30 ppm or less, and further preferably 10 ppm or less. Preferably, the magnesium alloy is substantially free from rare-earth elements and aluminum. Where an amount of an impurity element in the alloy is less than 1 ppm, it is regarded that the alloy is substantially free from the impurity element. The amount of impurity may be determined, for example, by ICP optical emission spectrometry.

### Production of Magnesium Alloy

In accordance with an ordinal production method of a magnesium alloy, the magnesium alloy may be produced by throwing ground metals or alloys of Mg, Zn, Zr, Mn into a crucible, melting the ground metals and/or alloys in the crucible at a temperature from 650 to 800 °C, and casting the molten alloy. Where necessary, the cast alloy may be subjected to solution heat treatment. The ground metals do not contain rare-earth elements (and aluminum). It is possible to suppress the amounts of Fe, Ni, Co, and Cu in the impurities by the use of high purity ground metals. Fe, Ni, and Co in the impurities may be removed by de-ironing treatment to the molten alloy. In addition, or alternatively, it is possible to use ground metals produced by distillation refining.

### Metal microstructure and mechanical properties

By the above-described controls of composition and production process, the magnesium alloy can have a fine and uniform structure as seen in a particle size distribution with an average crystal particle size from 1.0 to 3.0 µm (for example, from 1.0 to 2.0 µm) and a standard deviation of 0.7 µm or smaller (for example, from 0.5 to 0.7 µm). The standard deviation is preferably 0.65 µm or smaller. Fine precipitates containing Zr may each have a particle size smaller than 500 nm (preferably smaller than 100 nm). A matrix phase excluding the Zr precipitates may preferably be a single-phase solid solution of Mg-Zn-Mn ternary alloy.

The alloy has the following mechanical properties: a tensile strength from 230 to 380 MPa (for example, from 250 to 300 MPa), a proof strength from 145 to 220 MPa, and an elongation at breakage from 15 to 50% (for example, from 25 to 40%) in accordance with JIS Z2241. The alloy preferably has a tensile strength exceeding 280 MPa. The alloy preferably has an elongation at breakage exceeding 30%.

### Shape of Stent Scaffold

The ingots prepared in the above-described manner are subjected to hot extrusion to produce a magnesium alloy tubular material, and the thus-obtained magnesium alloy tubular material is laser-processed to form a stent scaffold (core structure).

The stent of the present invention may be formed into various scaffold shapes including conventional ones. For example, the stent may have the scaffold shape shown in Fig. 2 or Fig. 3.

### Electropolishing

As a pretreatment for forming a corrosion-resistant layer having a smooth surface, a preferable method of producing a core structure having an arbitrary size includes: connecting a laser-processed stent scaffold and a metal plate to an anode and a cathode, respectively, via a direct current (DC) power source in an electrolytic solution; and applying a voltage to them so as to electropolish the stent scaffold on the side of the anode.

### Formation of First Anticorrosive layer

In order to form a first anticorrosive layer, the core structure is subjected to fluorination. As long as a MgF₂ layer can be formed, the condition of fluorination is not particularly limited. For example, the core structure may be immersed in a treatment liquid such as an aqueous solution of hydrofluoric acid (HF) to carry out fluorination. It is preferable to shake the core structure at a speed of, for example, 50 to 200 rpm (preferably 80 to 150 rpm) during immersion. Then, the core structure on the surface of which the MgF₂ layer is formed is taken out and sufficiently washed with a cleaning solution (for example, acetone and water). The core structure is, for example, washed by ultrasonic cleaning. Where the core structure is dried after cleaning, the core structure is preferably dried at a temperature from 50 to 60°C under reduced pressure for 24 hours or longer. Further in order to form an anticorrosive layer with a smooth surface, the mirror-finished core structure obtained by electro-polishing may be subjected to fluorination.

### Feature of First Anticorrosive layer

The first anticorrosive layer of the stent according to the present invention contains magnesium fluoride as a main component. For example, the first anticorrosive layer may contain 90% or more of MgF₂ as a main component. The first anticorrosive layer may also contain oxides and hydroxides such as MgO and Mg(OH)₂ as accessary components. It should be noted that the first anticorrosive layer may also contain oxides and hydroxides of metals other than magnesium which constitute the stent.

### Layer Thickness of First Anticorrosive Layer

The first anticorrosive layer of the stent according to the present invention suitably has a layer thickness of 0.1 µm or larger (preferably 1 µm or larger) in order to exhibit corrosion resistance and a layer thickness of 3 µm or smaller, preferably 2 µm or smaller in order to exhibit deformation followability.

### Formation of Second Anticorrosive Layer

The diamond-carbon coating layer is formed by using a chemical vapor deposition (CVD) method.

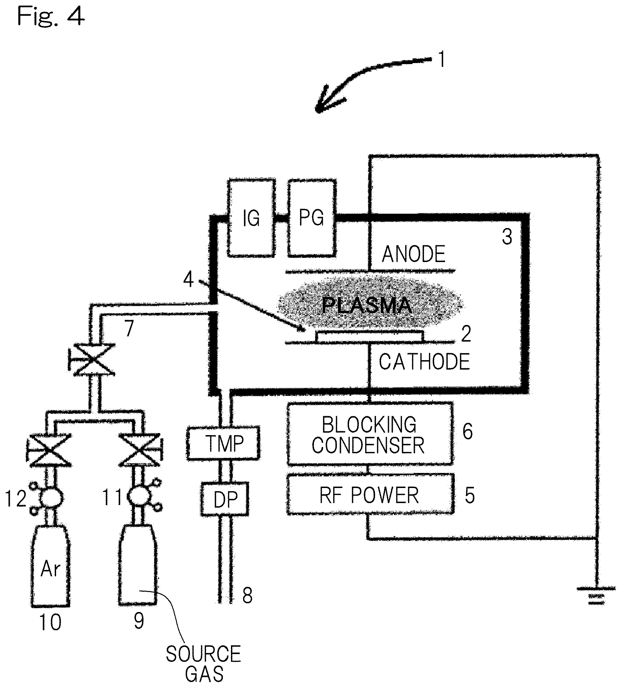

Fig. 4 is a schematic cross-sectional view of an apparatus used for forming a second anticorrosive layer, and shows a plasma CVD apparatus comprising a high frequency power source as discharge power source. The plasma CVD apparatus 1 is provided with a vacuum vessel 3 in which an electrode plate 2 that also serves as a substrate holder is installed at a lower portion. On the electrode plate 2 a core structure 4 coated with a first anticorrosive layer is placed. The electrode plate 2 is connected to a radio frequency (RF) power supply 5 and a blocking capacitor 6.

The vacuum vessel 3 is provided with a gas-introducing line 7 and a gas-exhausting port 8. The gas-introducing line 7 introduces a gas containing a carbon-containing gas [C-based gas such as acetylene] or a silicon- and carbon-containing gas [Si-C-based gas such as tetramethylsilane (TMS)], which is a source gas, and a bombard treatment gas (an inert gas such as Ar). The gas-exhausting port 8 is connected to an exhaust system (not shown). The gas-introducing line 7 is connected to a source gas supply device 9 and a bombard gas supply device 10 are connected to the gas-introducing line 7 via mass flow controllers 11 and 12, respectively. The vacuum vessel 3 is grounded.

The core structure 4 coated with the first anticorrosive layer is placed on the electrode plate 2, then the pressure inside of the vacuum vessel 3 is adjusted to a predetermined pressure by exhausting gas from the exhaust port 8 using an exhaust system (not shown). A C-based gas (for example, acetylene) or a Si-C-based gas (for example, tetramethylsilane), which is a source gas (raw material gas), is supplied from a source gas supply device 9, and the flow rate is adjusted using a mass flow controller 11 so as to be introduced into the vacuum vessel 3. During this time, high frequency (RF) is applied from the high frequency power source 5 to the electrode plate 2 to make the C-based gas or Si-C-based gas introduced into the vacuum vessel 3 into the plasma CVD apparatus.

By applying self-bias to the electrode plate 2 on which the core structure 4 coated with the first anticorrosive layer is placed, positive ions in the plasma apparatus are attracted to the core structure 4, so that a dense diamond-like thin film or a dense silicon-containing diamond-like thin film is locally formed on the surface of the core structure 4.

Specifically, a C-based gas containing carbon or a Si-C-based gas containing silicon and carbon used as a source gas is introduced into the chamber on which the base substrate is placed at a flow rate of 50 to 250 cm³/min (1 atm, 0°C), preferably 100 to 200 sccm, so as to give a pressure of 1 to 5 Pa, and a high frequency power of 100 to 500 W is applied to the RF electrode. Accordingly, a diamond-like carbon coat layer (DLC layer) or a silicon-containing diamond-like carbon coat layer (Si-DLC layer) is preferably formed.

Examples of the C-based gas containing carbon may include a gas containing acetylene, methane, and the like as main components. Examples of the Si-C-based gas containing silicon and carbon may include monomethylsilane, triethylsilane, tetramethylsilane and the like as main components. Alternatively, as the Si-C-based source gas, it may be used a mixture containing one or more of silicon-based gas containing at least silicon and one or more of carbon gas (alkane or the like).

Accordingly, the C-based gas (for example, acetylene) or the Si-C-based gas (for example, tetramethylsilane), as the source gas, is ionized by the plasma CVD method so as to form a DLC film or a silicon-containing DLC film on the surface of the core structure 4, resulting in a core structure (bioabsorbable stent) in which a second anticorrosive layer is formed on the first anticorrosive layer.

### Structure and Layer Thickness of Second Anticorrosive Layer

According to the present invention, by forming a second anticorrosive layer composed of a diamond-like carbon coat layer or a silicon-containing diamond-like carbon coat layer on the first anticorrosive layer, corrosion resistance of the Mg alloy can be significantly improved without deteriorating bioabsorption property of the stent structure. The second anticorrosive layer has a thickness of 10 nm to 5 µm, preferably 20 nm to 2 µm, and more preferably 20 nm to 500 nm. Too thin thickness may cause a tendency of insufficient anticorrosion effect, while too thick thickness may cause a tendency to inhibit bioabsorbability.

### Biodegradable Resin Layer

In the stent of the present invention, a cover layer comprising a biodegradable polymer and an intimal thickening inhibitor may be preferably formed on the entire surface or a part of the surface of the second anticorrosive layer. Examples of the biodegradable polymers may include polyesters, such as a poly-L-lactic acid (PLLA), a poly-D,L-lactic acid (PDLLA), a poly(lactic acid-glycolic acid) (PLGA), a polyglycolic acid (PGA), a poly caprolactone (PCL), a polylactic acid-ε-caprolactone (PLCL), a poly(glycolic acid-ε-caprolactone) (PGCL), a poly-p-dioxanone, a poly(glycolic acid-trimethylene carbonate), a poly-β-hydroxybutyric acid, and others.

### Intimal Thickening Inhibitor

Examples of the intimal thickening inhibitor may include sirolimus, everolimus, biolimus A9, zotarolimus, paclitaxel, etc.

### Performance of Stent

The stent on which the first and second anticorrosive layer having the smooth surface is formed as described above can have a significantly suppressed temporal reduction of a radial force in a simulated plasma solution (EMEM + 10% FBS) at 37°C under 5% COz atmosphere as well as in pig coronary arteries, when compared with a stent that does not fall within the scope of the present invention or a stent without an anticorrosive layer (core structure alone).

### Preparation of Magnesium Alloy

High purity ground metals of Mg, Zn, Mn, and Zr were prepared as initial materials. Each of the metals was weighed so as to have a component concentration as described in Table 1 and was thrown into a crucible. Then, at 730°C the metals were molten with stirring, and a thus-obtained melt was cast to form ingots. Thus-obtained magnesium alloys of Example 1 and Example 2 contained the main components at formulation ratios which fall within the present invention. The initial materials used did not contain rare earth elements and aluminum even as unavoidable impurities. In this regard, 99.99 % pure magnesium ground metal having a low concentration of impurity Cu was used. De-ironing treatment was carried out in the furnace in order to remove iron and nickel from the melt. Concentrations of impurities in the thus-obtained samples were determined using an ICP optical emission spectrometer (AGILENT 720 ICP-OES manufactured by AGILENT). Table 1 shows the compositions of Example 1 and Example 2. The concentrations of Fe, Ni, and Cu were all lower than 8 ppm (Ni and Cu were lower than 3 ppm). Al and the rare-earth elements were not detected, and Co was also below a detection limit. The total content of the unavoidable impurities was 11 or 12 ppm.

**[Table 1]**

| | Component concentration (%) | | | | Impurity concentration (ppm) | | | |
|---|---|---|---|---|---|---|---|---|
| | Mg | Zn | Mn | I Zr | Fe | Ni | Cu | Total |
| Production Example 1 | the balance | 1.86 | 0.14 | 0.12 | 5 | 3 | 3 | 11 |
| Production Example 2 | the balance | 0.95 | 0.11 | 0.24 | 8 | 3 | 1 | 12 |

### Measurement of Mechanical Properties

Each alloy according to the examples was formed into a round bar material through hot extrusion. In accordance with JIS Z2241, a tensile strength, a proof strength, and an elongation at breakage of the round bar material were determined. Table 2 shows the results.

**[Table 2]**

| | Tensile strength (MPa) | Proof strength (MPa) | Elongation (%) | Average crystal particle size (µm) | Standard deviation |
|---|---|---|---|---|---|
| Production Example 1 | 288 | 213 | 38 | 1.97 | 0.62 |
| Production Example 2 | 297 | 217 | 27 | 1.97 | 0.63 |

### EXAMPLE

Hereinafter, the present invention will be specifically described with reference to Examples. The present invention, however, is not limited to the following Examples.

### Evaluation of Anticorrosive property

A stent sample obtained in Examples and Comparative Examples described below was immersed in a 37°C simulated plasma solution (EMEM + 10% FBS), then was uniformly expanded to have an inner diameter of 3 mm, and was shaken at 100 rpm with keeping immersion at 37°C under 5% COz atmosphere. The sample was taken out at 28 days after immersion, and the radial force of the sample was measured (n = 4). Further, the sample was cleaned ultrasonically with tetrahydrofuran (THF) and chromic acid solution to completely remove coating polymers and corrosion products such as magnesium hydroxide, etc., and the weight change of the core structure was evaluated (n = 5). As to the radial force measurement, RX550/650 (produced by Machine Solutions Inc.) was used.

### Example 1

A core structure comprising the above-described stent scaffold formed from the magnesium alloy obtained in the Production Example 1 was immersed in a 27 M hydrofluoric-acid aqueous solution (2 mL) and reciprocally moved at a rate of 100 rpm. Then, the stent was taken out after 24 hours, and subjected to ultrasonic cleaning sufficiently with water and acetone followed by drying the core structure for 24 hours at 60°C under vacuum to prepare a core structure on which a first corrosion resistant layer (thickness: 1 µm) was formed. A diamond-like carbon coat layer having a thickness of 50 nm was formed on this structure so as to form a second corrosion resistant layer. Onto a surface of thus-obtained structure, a first cover layer containing 400 µg of a first polymer PCL was spray-coated, and then a second cover layer containing 150 µg of a second polymer PDLLA and 100 µg of sirolimus was spray-coated so as to obtain a stent sample shown in Fig. 1.

### Comparative Example 1

Onto a surface of a core structure having the stent scaffold (without fluorination), a first cover layer containing 400 µg of a first polymer PCL was spray-coated, and then a second cover layer containing 150 µg of a second polymer PDLLA and 100 µg of sirolimus was spray-coated so as to obtain a stent sample.

### Comparative Example 2

A core structure comprising the above-described stent scaffold was immersed in a 27 M hydrofluoric-acid aqueous solution (2 mL) and reciprocally moved at a rate of 100 rpm. Then, the stent was taken out after 24 hours, and subjected to ultrasonic cleaning sufficiently with water and acetone followed by drying the core structure for 24 hours at 60°C under vacuum to prepare a core structure on which a first corrosion resistant layer (thickness: 1 µm) was formed. Onto a surface of thus-obtained structure, a first cover layer containing 400 µg of a first polymer PCL was spray-coated, and then a second cover layer containing 150 µg of a second polymer PDLLA and 100 µg of sirolimus was spray-coated so as to obtain a stent sample.

**Table 3**

| Components of stent samples in Example 1 and Comparative Examples 1 to 2 | | | | | |
|---|---|---|---|---|---|
| | Core Structure | First Anticorrosive Layer | Second Anticorrosive Layer | First Coating Polymer Layer | Second Coating Polymer Layer |
| Example 1 | Mg alloy | Magnesium fluoride | DLC | PCL | PDLLA/Sirolimus |
| | 100 µm | 1 µm | 50 nm | 400 µg | 150 µg/100 µg |
| Comparative Example 1 | Mg alloy | None | None | PCL | PDLLA/Sirolimus |
| | 100 µm | | | 400 µg | 150 µg/100 µg |
| Comparative Example 2 | Mg alloy | Magnesium fluoride | None | PCL | PDLLA/Sirolimus |
| | 100 µm | 1 µm | | 400 µg | 150 µg/100 µg |

### Weight Change of Core Structure Before and After Immersion

The core structure weights of each of the samples before immersion as well as after immersion for 28 days in the simulated plasma solution were measured. Table 4 shows the result of the weight residual ratio of the core structure before and after immersion calculated based on the weight of the core structure before immersion. The weight of the core structure before immersion was 6.13 mg.

**Table 4**

| Weight Change of Core Structure Before and After Immersion (Weight Residual Ratio [%]) | | |
|---|---|---|
| | Before immersion | After immersion for 28 days |
| Example 1 | 100 | 98.0 ± 3.5 |
| Com. Ex. 1 | 100 | 80.1 ± 5.7 |
| Com. Ex. 2 | 100 | 90.1 ± 4.3 |

### Relative Evaluation of Weight Change after Immersion for 28 Days

The sample (Comparative Example 2) with the magnesium fluoride layer as the first anticorrosive layer had higher weight residual ratio than the comparative sample (Comparative Example 1) without the anticorrosive layer. Further, it was confirmed that the sample (Example 1) comprising the diamond-like carbon coat layer as the second anticorrosive layer in addition to the first anticorrosive layer had further increase in weight residual ratio. That is, the weight residual ratio was higher in the order of Example 1> Comparative Example 2 > Comparative Example 1.

### Change in Radial Force of Core Structure Before and After Immersion

The core structure radial force of each of the samples before immersion as well as after immersion for 28 days in a simulated plasma solution was measured. Table 8 shows the result of the radial force residual ratio of the core structure before and after immersion calculated based on the radial force of the core structure before immersion. The radial force of the core structure before immersion was 65.4 N/mm.

**Table 5**

| Change in Physical Properties of Core Structure Before and After Immersion (Radial force residual ratio [%]) | | |
|---|---|---|
| | Before immersion | After immersion for 28 days |
| Example 1 | 100 | 94.0 ± 4.3 |
| Com. Ex. 1 | 100 | 23.5 ± 6.7 |
| Com. Ex. 2 | 100 | 89.1 ± 4.5 |

### Relative Evaluation of Radial Force at After immersion for 28 days

It was confirmed that the sample (Example 1) comprising the diamond-like carbon coat layer as the second anticorrosive layer in addition to the first anticorrosive layer had the highest radial force residual ratio, followed by the sample (Comparative Example 2) with the magnesium fluoride layer as the first anticorrosive layer. As is the case of the weight residual ratio, the radial force residual ratio was higher in the order of Example 1> Comparative Example 2 > Comparative Example 1. That is, it was clarified that the corrosion was suppressed by using two anticorrosive layers.

### Example 2

A core structure comprising the above-described stent scaffold formed from the magnesium alloy obtained in the Production Example 1 was immersed in a 27 M hydrofluoric-acid aqueous solution (2 mL) and reciprocally moved at a rate of 100 rpm. Then, the stent was taken out after 24 hours, and subjected to ultrasonic cleaning sufficiently with water and acetone followed by drying the core structure for 24 hours at 60°C under vacuum to prepare a core structure on which a first corrosion resistant layer (thickness: 1 µm) was formed. This structure was placed in the plasma CVD apparatus shown in Fig. 4, and then tetramethylsilane was introduced as a source gas using the apparatus shown in Fig. 4 to form a silicon-containing diamond-like carbon coat layer as a second corrosion resistant layer having a thickness of 50 nm on this structure. Onto a surface of thus-obtained structure, a first cover layer containing 400 µg of a first polymer PCL was spray-coated, and then a second cover layer containing 150 µg of a second polymer PDLLA and 100 µg of sirolimus was spray-coated so as to obtain a stent sample shown in Fig. 1.

### Comparative Example 3

Onto a surface of a core structure having the stent scaffold (without fluorination), a first cover layer containing 400 µg of a first polymer PCL was spray-coated, and then a second cover layer containing 150 µg of a second polymer PDLLA and 100 µg of sirolimus was spray-coated so as to obtain a stent sample.

### Comparative Example 4

A core structure comprising the above-described stent scaffold was immersed in a 27 M hydrofluoric-acid aqueous solution (2 mL) and reciprocally moved at a rate of 100 rpm. Then, the stent was taken out after 24 hours, and subjected to ultrasonic cleaning sufficiently with water and acetone followed by drying the core structure for 24 hours at 60°C under vacuum to prepare a core structure on which a first corrosion resistant layer (thickness: 1 µm) was formed. Onto a surface of thus-obtained structure, a first cover layer containing 400 µg of a first polymer PCL was spray-coated, and then a second cover layer containing 150 µg of a second polymer PDLLA and 100 µg of sirolimus was spray-coated so as to obtain a stent sample.

**Table 6**

| Components of stent samples in Example 2 and Comparative Examples 3 to 4 | | | | | |
|---|---|---|---|---|---|
| | Core Structure | First Anticorrosive Layer | Second Anticorrosive Layer | First Coating Polymer Layer | Second Coating Polymer Layer |
| Example 2 | Mg alloy | Magnesium fluoride | Si-containing DLC | PCL | PDLLA/Sirolimus |
| | 100 µm | 1 µm | 50 nm | 400 µg | 150 /100 µg |
| Comparative Example 3 | Mg alloy | None | None | PCL | PDLLA/Sirolimus |
| | 100 µm | | | 400 µg | 150 µg/100 µg |
| Comparative Example 4 | Mg alloy | Magnesium fluoride | None | PCL | PDLLA/Sirolimus |
| | 100 µm | 1 µm | | 400 µg | 150 gg/100 µg |

### Weight Change of Core Structure Before and After Immersion

The core structure weights of each of the samples before immersion as well as after immersion for 28 days in the simulated plasma solution were measured. Table 7 shows the result of the weight residual ratio of the core structure before and after immersion calculated based on the weight of the core structure before immersion. The weight of the core structure before immersion was 6.13 mg.

**Table 7**

| Weight Change of Core Structure Before and After Immersion (Weight Residual Ratio [%]) | | |
|---|---|---|
| | Before immersion | After immersion for 28 days |
| Example 2 | 100 | 98.0 ± 3.5 |
| Com. Ex. 3 | 100 | 80.1 ± 5.7 |
| Com. Ex. 4 | 100 | 90.1 ± 4.3 |

### Relative Evaluation of Weight Change after Immersion for 28 Days

The sample (Comparative Example 4) with the magnesium fluoride layer as the first anticorrosive layer had higher weight residual ratio than the comparative sample (Comparative Example 3) without the anticorrosive layer. Further, it was confirmed that the sample (Example 2) comprising the silicon-containing diamond-like carbon coat layer as the second anticorrosive layer in addition to the first anticorrosive layer had further increase in weight residual ratio. That is, the weight residual ratio was higher in the order of Example 2 > Comparative Example 4 > Comparative Example 3.

### Change in Radial Force of Core Structure Before and After Immersion

The core structure radial force of each of the samples before immersion as well as after immersion for 28 days in a simulated plasma solution was measured. Table 8 shows the result of the radial force residual ratio of the core structure before and after immersion calculated based on the radial force of the core structure before immersion. The radial force of the core structure before immersion was 65.4 N/mm.

**Table 8**

| Change in Physical Properties of Core Structure Before and After Immersion (Radial force residual ratio [%]) | | |
|---|---|---|
| | Before immersion | After immersion for 28 days |
| Example 2 | 100 | 98.0 ± 3.5 |
| Com. Ex. 3 | 100 | 23.5 ± 6.7 |
| Com. Ex. 4 | 100 | 89.1 ± 4.5 |

### Relative Evaluation of Radial Force After Immersion for 28 Days

It was confirmed that the sample (Example 2) comprising the silicon-containing diamond-like carbon coat layer as the second anticorrosive layer in addition to the first anticorrosive layer had the highest radial force residual ratio, followed by the sample (Comparative Example 4) with the magnesium fluoride layer as the first anticorrosive layer. Likewise, the weight residual ratio, the radial force residual ratio was higher in the order of Example 2 > Comparative Example 4 > Comparative Example 2. That is, it was clarified that the corrosion was suppressed by using two anticorrosive layers.

### INDUSTRIAL APPLICABILITY

The present invention can provide a stent comprising a first corrosion resistant layer and a second corrosion resistant layer that effectively delay decrease in mechanical strength associated with accelerated corrosion of the core structure. Therefore, the present invention contributes to development of medical technology and thus has remarkable industrial applicability.

### REFERENCE NUMERALS

- **a**: Core structure (Mg alloy)
- **b**: First anticorrosive layer (magnesium fluoride layer)
- **c**: Second anticorrosive layer (DLC layer or Si-DLC layer)
- **d**: Biodegradable resin layer
- e: Biodegradable resin layer (containing a medicine)
- 1: Apparatus used for forming a second anticorrosive layer
- 2: Electrode plate
- 3: Vacuum vessel
- 4: Core structure comprising the first anticorrosive layer
- 5: RF (high frequency) power supply
- 6: Blocking condenser
- 7: Gas-introducing line
- 8: Gas-exhausting port
- 9: Source gas supply device
- 10: Bombard gas supply device
- 11: Mass flow controller
- 12: Mass flow controller

## Claims

1. A bioabsorbable stent comprising a core structure of a magnesium alloy, a first anticorrosive layer containing magnesium fluoride as a main component formed on the core structure, and
a second anticorrosive layer of a carbon-coated layer containing a diamond-like carbon on the first anticorrosive layer,
the first anticorrosive layer has a layer thickness of 0.1 to 3 µm and the second anticorrosive layer has a layer thickness of 10 nm to 5 µm.

2. The bioabsorbable stent according to claim 1, wherein the magnesium alloy contains, in % by mass, 0.95 to 2.00% of Zn, 0.05% to 0.30% of Zr, and 0.05 to 0.20% of Mn, and the balance consisting of Mg and unavoidable impurities, and has a grain size distribution with an average crystal grain size from 1.0 to 3.0 µm and a standard deviation of 0.7 µm or lower.

3. The bioabsorbable stent according to claim 1 or 2, wherein the first anticorrosive layer is formed by fluorination of a surface of the magnesium alloy.

4. The bioabsorbable stent according to any one of claims 1 to 3, wherein the diamond-like carbon of the second anticorrosive layer is a silicon-containing diamond-like carbon.

5. The bioabsorbable stent according to any one of claims 1 to 4, wherein a biodegradable polymer layer is formed on at least a part of the surface of the second anticorrosive layer.

6. The bioabsorbable stent according to claim 5, wherein the biodegradable polymer layer contains an intimal thickening inhibitor.

7. The bioabsorbable stent according to claim 6, wherein the intimal thickening inhibitor is a limus type drug.

8. A method for producing a bioabsorbable stent comprising a magnesium alloy containing
(1) fluorinating a surface of a core structure made of a magnesium alloy, to form a first anticorrosive layer containing magnesium fluoride as a main component and having a layer thickness of 0.1 to 3 µm, and then,
(2) subjecting the core structure with the first anticorrosive layer to be placed in a high-frequency plasma CVD apparatus such that a diamond-like carbon film is coated on the core structure via introduction of a carbon-containing source gas so as to form a second anticorrosive layer having a layer thickness of 10 nm to 5 µm.

9. The production method according to claim 8, wherein a source gas containing carbon and silicon is introduced as the source gas, so that the surface of the core structure is coated with a silicon-containing diamond-like carbon film to form the second anticorrosive layer.

## Patentansprüche

1. Bioabsorbierbarer Stent, umfassend eine Kernstruktur aus einer Magnesiumlegierung, eine erste antikorrosive Schicht, die Magnesiumfluorid als Hauptkomponente enthält, die auf der Kernstruktur gebildet ist, und
eine zweite antikorrosive Schicht einer kohlenstoffbeschichteten Schicht, die einen diamantartigen Kohlenstoff auf der ersten antikorrosiven Schicht enthält,
wobei die erste antikorrosive Schicht eine Schichtdicke von 0,1 bis 3 µm hat und die zweite antikorrosive Schicht eine Schichtdicke von 10 nm bis 5 µm hat.

2. Bioabsorbierbarer Stent nach Anspruch 1, wobei die Magnesiumlegierung in Massen% 0,95 bis 2,00 % Zn, 0,05 % bis 0,30 % Zr und 0,05 bis 0,20 % Mn enthält und der Rest aus Mg und unvermeidbaren Verunreinigungen besteht, und eine Korngrößenverteilung mit einer durchschnittlichen Kristallkorngröße von 1,0 bis 3,0 µm und einer Standardabweichung von 0,7 µm oder niedriger hat.

3. Bioabsorbierbarer Stent nach Anspruch 1 oder 2, wobei die erste antikorrosive Schicht durch Fluorierung einer Oberfläche der Magnesiumlegierung gebildet wird.

4. Bioabsorbierbarer Stent nach einem der Ansprüche 1 bis 3, wobei der diamantartige Kohlenstoff der zweiten antikorrosiven Schicht ein siliciumhaltiger diamantartiger Kohlenstoff ist.

5. Bioabsorbierbarer Stent nach einem der Ansprüche 1 bis 4, wobei eine biologisch abbaubare Polymerschicht auf mindestens einem Teil der Oberfläche der zweiten antikorrosiven Schicht gebildet ist.

6. Bioabsorbierbarer Stent nach Anspruch 5, wobei die biologisch abbaubare Polymerschicht einen Intimaverdickungsinhibitor enthält.

7. Bioabsorbierbarer Stent nach Anspruch 6, wobei der Intimaverdickungsinhibitor ein Arzneimittel vom Limus-Typ ist.

8. Verfahren zum Produzieren eines bioabsorbierbaren Stents, der eine Magnesiumlegierung umfasst, enthaltend:
(1) Fluorieren einer Oberfläche einer Kernstruktur, die aus einer Magnesiumlegierung gefertigt ist, um eine erste antikorrosive Schicht zu bilden, die Magnesiumfluorid als Hauptkomponente enthält und eine Schichtdicke von 0,1 bis 3 µm aufweist, und dann
(2) Aussetzen der Kernstruktur mit der ersten antikorrosiven Schicht Platzierung in einem Hochfrequenz-Plasma-CVD-Apparat, so dass ein Film aus diamantartigem Kohlenstoff mittels Einbringung eines kohlenstoffhaltigen Quellgases als Beschichtung auf die Kernstruktur aufgebracht wird, um so eine zweite antikorrosive Schicht mit einer Schichtdicke von 10 nm bis 5 µm zu bilden.

9. Produktionsverfahren nach Anspruch 8, wobei ein Quellgas, das Kohlenstoff und Silicium enthält, als Quellgas eingebracht wird, so dass die Oberfläche der Kernstruktur mit einem Film aus siliciumhaltigen diamantartigem Kohlenstoff beschichtet wird, um die zweite antikorrosive Schicht zu bilden.

## Revendications

1. Un stent bioabsorbable comprenant une structure centrale en alliage de magnésium, une première couche anticorrosive contenant du fluorure de magnésium comme composant principal formée sur la structure centrale, et
une seconde couche anticorrosive constituée d'une couche de carbone contenant un carbone de type diamant sur la première couche anticorrosive,
la première couche anticorrosive a une épaisseur de 0,1 à 3 µm et la seconde couche anticorrosive a une épaisseur de 10 nm à 5 µm.

2. Le stent bioabsorbable selon la revendication 1, dans lequel l'alliage de magnésium contient, en % de masse, de 0,95 à 2,00 % de Zn, de 0,05 à 0,30 % de Zr, et de 0,05 à 0,20 % de Mn, le reste étant constitué de Mg et d'impuretés inévitables, et a une distribution de taille de grain avec une taille moyenne de grain cristallin de 1,0 à 3,0 µm et une déviation standard de 0,7 µm ou moins.

3. Le stent bioabsorbable selon la revendication 1 ou 2, dans lequel la première couche anticorrosive est formée par la fluoration d'une surface de l'alliage de magnésium.

4. Le stent bioabsorbable selon l'une des revendications 1 à 3, dans lequel le carbone de type diamant de la seconde couche anticorrosive est un carbone de type diamant contenant du silicium.

5. Le stent bioabsorbable selon l'une des revendications 1 à 4, dans lequel une couche de polymère biodégradable est formée sur au moins une partie de la surface de la seconde couche anticorrosive.

6. Le stent bioabsorbable selon la revendication 5, dans lequel la couche de polymère biodégradable contient un inhibiteur d'épaississement intimal.

7. Le stent bioabsorbable selon la revendication 6, dans lequel l'inhibiteur de l'épaississement intimal est un médicament de type limus.

8. Procédé de fabrication d'un stent bioabsorbable comprenant un alliage de magnésium contenant
(1) fluorer une surface d'une structure centrale faite d'un alliage de magnésium, pour former une première couche anticorrosive contenant du fluorure de magnésium comme composant principal et ayant une épaisseur de couche de 0,1 à 3 µm, et ensuite,
(2) placer la structure centrale avec la première couche anticorrosive dans un appareil de dépôt en phase vapeur par plasma à haute fréquence de manière à ce qu'un film de carbone de type diamant soit déposé sur la structure centrale par l'introduction d'un gaz source contenant du carbone afin de former une deuxième couche anticorrosive d'une épaisseur comprise entre 10 nm et 5 µm.

9. Procédé de fabrication selon la revendication 8, dans lequel un gaz source contenant du carbone et du silicium est introduit comme gaz source, de sorte que la surface de la structure centrale est recouverte d'un film de carbone de type diamant contenant du silicium pour former la seconde couche anticorrosive.
